# EUROPEAN PATENT APPLICATION

(11) **EP 3 168 600 A1**
(43) Date of publication of application: **17.05.2017**
(21) Application number: 15818474.7
(22) Date of filing: 02.07.2015
(51) Int. Cl.: G01N 21/27, G01N 21/359

(54) **BIOLOGICAL COMPONENT INFORMATION MEASUREMENT DEVICE**

(30) Priority: 08.07.2014 JP 2014140395; 28.01.2015 JP 2015014302
(71) Applicant: Mitsumi Electric Co., Ltd., Tama-Shi, Tokyo 206-8567 (JP)
(72) Inventor: SATO, Masahiro, Tokyo 206-8567 (JP); MURAYAMA, Noriyoshi, Tokyo 206-8567 (JP); SAITO, Masahiro, Tokyo 206-8567 (JP); MATSUDA, Hiroshi, Tokyo 206-8567 (JP); NARISAWA, Nozomu, Tokyo 206-8567 (JP)
(74) Representative: Epping - Hermann - Fischer
(86) International application number: PCT/JP2015/003327
(87) International publication number: WO 2016/006211

(57) **Abstract**

In the present invention, without decreasing measurement accuracy, a biological component information measurement device can have a miniaturized device structure. In a biological component information measurement device (100), a sample container (104) accommodates a measurement target (105) such as blood, cultured cells, or urine, and a light from a light source (101) is separated into spectral components using a rotating diffraction grating (110) and caused to be incident on the measurement target (105). Due to this configuration, it is possible to reduce the number of parts of a spectral optical system and the amount of space required therefor. As a result, it is possible to, in particular, miniaturize the spectral optical system without decreasing measurement accuracy.

## Description

### Technical Field

The present invention relates to a biological component information measurement device which measures information of a biological component such as a blood component and a urine component by use of light.

### Background Art

Conventionally, devices are known which measure the blood component by irradiating a sample (human body) with a near infrared ray, and analyzing reflection light from the sample. Such devices are disclosed in PTLS 1 to 4, for example.

In general, such devices include a first optical system that guides light from the light source to the measurement target, a second optical system that guides the light reflected by the measurement target, an optical system that separates the reflection light guided by the second optical system, a photodetector that receives the separated light, and a reference signal optical system for obtaining the reference signal for calibration.

In addition, a method is widely used in which a sample is put in an analysis cell, and the biological component is analyzed in a spectral manner. Examples of such a method include UV-Vis-NIR spectrophotometers available from Shimadzu Corporation, and spectrophotometers available from Hitachi High-Technologies Corporation.

In addition, conventionally, a method of measuring the urine component by use of light is disclosed in PTL 5 and the like, for example. In the method disclosed in PTL 5, the urine sample is irradiated with visible light or near infrared light, and the light absorbance at the wavelengths corresponding to the urine components to be measured is measured to simultaneously perform quantitative analysis of the urine components.

Advantageously, such a method of measuring the urine component using light can be implemented without using consumables such as reagent, test paper or the like unlike other methods using reagent, test paper, chemical light emission, or the like, and does not require complicated procedures.

### Citation List

### Patent Literature

PTL 1
   Japanese Patent Application Laid-Open No. 2006-87913
PTL2
   Japanese Patent Application Laid-Open No. 2002-65465
PTL3
   Japanese Patent Application Laid-Open No. 2007-259967
PTL4
   Japanese Patent Application Laid-Open No. 2012-191969
PTL5
   Japanese Patent Application Laid-Open No. 7-294519

### Summary of Invention

### Technical Problem

In the conventional biological component information measurement devices disclosed in PTLS 1 to 4, however, the photodetector, which is a principal component, is composed of an array type sensor, and as such there is a room for improvement in terms of downsizing and cost reduction.

In addition, in the method disclosed in PTL 5, a plurality of light sources (see FIGS. 2A to 2C of PTL 5), or, a light separation part (see FIGS. 5A to 5D of PTL 5) is provided for the purpose of obtaining light for irradiating the sample. Disadvantageously, when a plurality of light sources are provided, the configuration is accordingly complicated, and downsizing of the device is limited. In addition, when the light separation part is provided, a plurality of filters are required as the components of the light separation part, and downsizing of the device and cost reduction are limited.

In view of the foregoing, an object of the present invention is to provide a biological component information measurement device which can achieve downsizing of the device configuration without reducing the measurement accuracy.

### Solution to Problem

A biological component information measurement device of a mode of the present invention includes: a light source; a measurement target placement part in which a measurement target is disposed, in which light from the light source passes through the measurement target and is emitted therefrom; a photodetector configured to receive light passed through the measurement target; and a rotation diffraction grating disposed on an optical path from the light source to the measurement target, or an optical path from the measurement target to the photodetector, the rotation diffraction grating being configured to separate the light from the light source such that the light is incident on the measurement target, or separate the light passed through the measurement target such that the light is incident on the photodetector.

### Advantageous Effects of Invention

According to the present invention, it is possible to achieve a biological component information measurement device which can achieve downsizing of the device configuration without reducing the measurement accuracy.

In addition, for example, quantitative analysis of urine component is achieved without requiring reagent or test paper, and the size of the device can be reduced, and therefore, urine inspection can be simply performed at any place. As a result, kidney function or lever function can be determined on a daily basis, which helps health maintenance.

### Brief Description of Drawings

FIG. 1 is a schematic view illustrating a general configuration of a biological component information measurement device according to an embodiment;
FIGS. 2A to 2C are used for describing a diffraction operation of a rotation diffraction grating;
FIG. 3 is a plan view illustrating an external appearance of a MEMS device provided with the rotation diffraction grating;
FIGS. 4A and 4B illustrate variation of the signal size which is measured by a photodetector (PD) in the case where the position of the rotation diffraction grating is changed in the direction perpendicular to the mirror surface without changing the rotation position of the rotation diffraction grating;
FIGS. 5A to 5D are used for describing lock-in amplifier detection;
FIG. 6 is a schematic view illustrating a general configuration of a biological component information measurement device according to another embodiment;
FIG. 7 is a perspective view illustrating a detailed configuration of the biological component information measurement device;
FIG. 8 illustrates a spectrum of the rotation diffraction grating of the embodiment;
FIG. 9 illustrates a light source output in the case where a light source is pulse driven;
FIG. 10 is a schematic view illustrating a general configuration of the biological component information measurement device according to another embodiment; and
FIG. 11 is used for describing an exemplary use of the biological component information measurement device according to the embodiment.

### Description of Embodiments

In the following, embodiments of the present invention are described in detail with reference to the accompanying drawings.

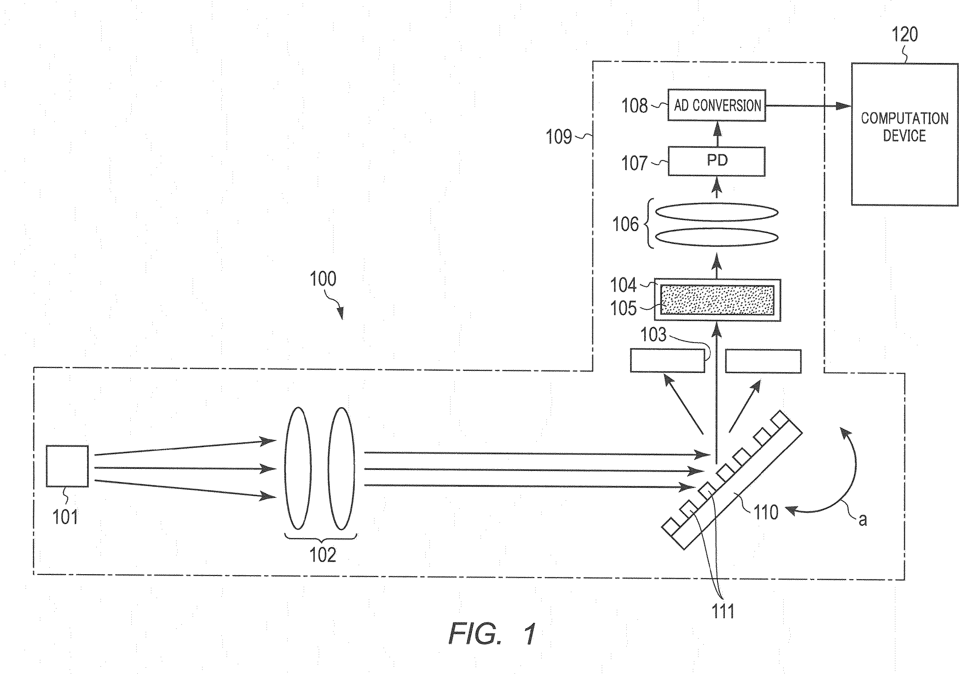

FIG. 1 is a schematic view illustrating a general configuration of biological component information measurement device 100 according to the embodiment of the present invention.

In biological component information measurement device 100, light from light source 101 is incident on rotation diffraction grating 110 through optical system 102. Light source 101 is composed of a light emitting diode (LED), a halogen lamp or a xenon lamp. Rotation diffraction grating 110 turns as illustrated with the arrow in the figure. The incidence surface of rotation diffraction grating 110 is a mirror surface which reflects incident light. That is, rotation diffraction grating 110 turns such that the incident angle to the mirror surface is changed. Rotation diffraction grating 110 reflects light having a wavelength corresponding to the rotation angle in a direction toward slit 103 to separate the incident light.

Light separated by rotation diffraction grating 110 is incident on sample container 104. Sample container 104 is a transparent container formed of quartz, glass or the like, and blood, cultured cells, urine and the like are stored in sample container 104 as measurement target 105. Light having passed through sample container 104 and measurement target 105 stored in sample container 104 is incident on PD 107 through optical system 106. A light reception signal obtained through photoelectric conversion of PD 107 is output to computation device 120 through analog digital conversion circuit (A/D conversion) 108. Computation device 120 is a device having an analysis program such as a personal computer and a smartphone, and obtains information of biological components such as a blood component and a urine component from the light reception signal by executing the analysis program. It is to be noted that all of the optical systems of biological component information measurement device 100 are housed in case 109.

For example, in the case of measurement of a urine component, computation device 120 calculates an transmission spectrum and an absorption spectrum from a signal detected for each wavelength, and performs the spectrum analysis for quantitative analysis of a urine component containing glucose, creatinine, bilirubin, an urea nitrogen, albumin, a ketone body, sodium chloride, occult blood, nitrites, urobilinogen and the like. Known methods such as the method disclosed in PTL 5, for example, may be used as the way of the spectrum analysis, and therefore detailed description thereof is omitted.

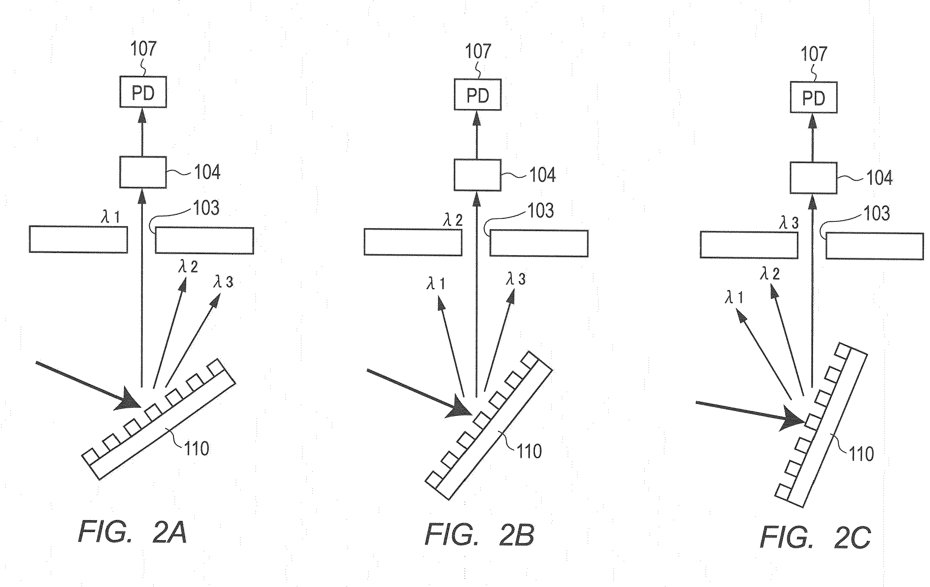

FIGS. 2A to 2C illustrate a diffraction operation of rotation diffraction grating 110. It is to be noted that optical system 106 is omitted in FIGS. 2A to 2C. At the rotation position illustrated in FIG. 2A, rotation diffraction grating 110 reflects a λ1 component of incident light in a direction toward slit 103 such that the λ1 component enters sample container 104. In addition, at the rotation position illustrated in FIG. 2B, rotation diffraction grating 110 reflects a λ2 component of the incident light in a direction toward slit 103 such that the λ2 component enters sample container 104. Further, at the rotation position illustrated in FIG. 2C, rotation diffraction grating 110 reflects a λ3 component of the incident light in a direction toward slit 103 such that the λ3 component enters sample container 104. In this manner, rotation diffraction grating 110 emits the light having a wavelength corresponding to the rotation angle to thereby separate the incident light.

In the present embodiment, light is separated using rotation diffraction grating 110, and thus a photodetector having a single light reception surface, not an array sensor, can be used as photodetector (PD) 107 unlike the case where a fixed diffraction grating is used. As a result, photodetector 107 having a simple configuration can be used, and accordingly, the cost can be reduced. In addition, unlike the case where a fixed diffraction grating is used, it is not necessary to provide a space for separating the light between the diffraction grating and photodetector 107, and accordingly, the size of the device can be reduced.

Here, in diffraction grating 110 of the present embodiment rotation, the movable portion of the micro electro mechanical system (MEMS) is the mirror surface, and the diffraction grating is formed on the mirror surface. That is, in rotation diffraction grating 110, a grating is formed on the mirror surface of the MEMS mirror.

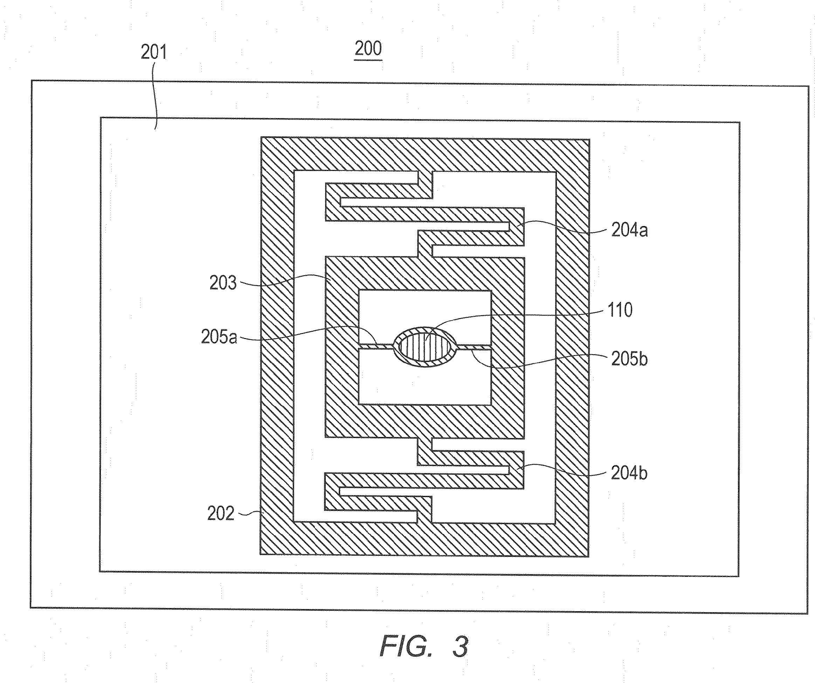

FIG. 3 is a plan view illustrating an external appearance of MEMS device 200 provided with rotation diffraction grating 110. MEMS device 200 includes driving section 201 composed of a driving circuit, an actuator and the like, rotation diffraction grating 110, fixation frame 202, movable frame 203, and beam parts 204 and 205. Driving section 201 has a function of driving rotation diffraction grating 110. In addition, driving section 201 includes fixation frame 202 and serves also as the base of rotation diffraction grating 110. Beam part 204 is composed of two beams 204a and 204b. Two beams 204a and 204b are provided to extend between two opposite edges of movable frame 203 and fixation frame 202. With this configuration, movable frame 203 is suspended with fixation frame 202 by beams 204a and 204b. In addition, beam part 205 is composed of two beams 205a and 205b. Two beams 205a and 205b are provided to extend between two opposite edges of rotation diffraction grating 110 and movable frame 203. With this configuration, rotation diffraction grating 110 is suspended with movable frame 203 by beams 205a and 205b.

Rotation diffraction grating 110 rotates when beams 204a and 204b are driven by driving section 201. To be more specific, when the left and the right of beams 204a and 204b are changed by driving section 201 in a staggered manner in the depth direction of the drawing, rotation diffraction grating 110 is driven into rotation in a predetermined angle range. Meanwhile, rotation diffraction grating 110 is driven into rotation at a rotational speed of 1 to 100 [Hz]. It should be noted that the rotational speed is not limited to this. The rotational speed may be set in accordance with the arithmetic speed of computation device 120 and the like. Rotation diffraction grating 110 may be driven by piezoelectric methods, electrostatic methods, electromagnetic driving methods, and the like.

The surface of rotation diffraction grating 110 is a mirror surface, and further, diffraction grating 111 is formed on the mirror surface. Diffraction grating 111 is parallel to the rotation axis of beams 204a and 204b. In the present embodiment, the pitch of diffraction grating 111 is 0.1 to 4 [µm]. In addition, the depth of diffraction grating 111 is 0.01 to 4 [µm]. With this configuration, rotation diffraction grating 110 can favorably separate a near infrared ray with the rotation. In the case where the measurement is performed by use of light other than near infrared rays, it suffices to select the pitch and/or the depth of diffraction grating 111 in accordance with the light.

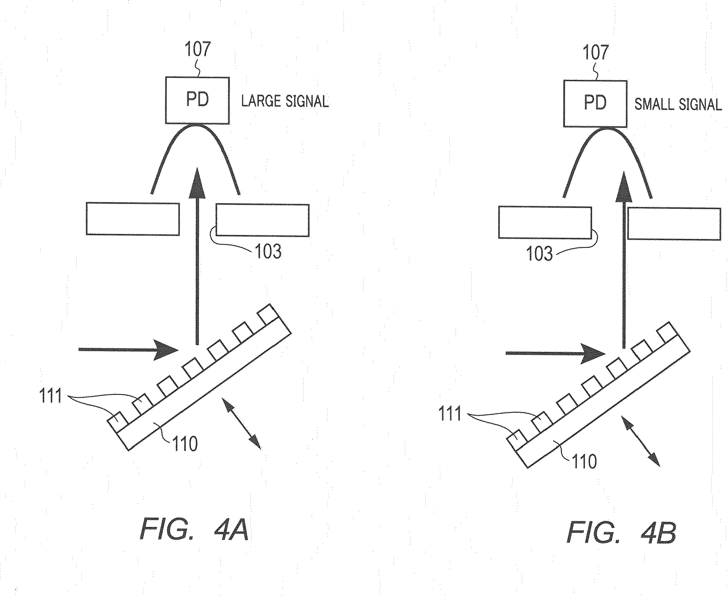

Further, in the present embodiment, rotation diffraction grating 110 is driven also in the direction perpendicular to the mirror surface as illustrated in FIG. 4. It is to be noted that sample container 104 and optical system 106 are omitted in FIG. 4. To be more specific, when beams 205a and 205b are simultaneously deflected by driving section 201 in the same depth direction of the drawing, rotation diffraction grating 110 is driven in the direction perpendicular to the mirror surface. For example, rotation diffraction grating 110 is driven into a high-frequency simple harmonic motion of several 10 [KHz] in the direction perpendicular to the mirror surface. FIG. 4A and FIG. 4B illustrate variation of the signal size measured by PD 107 in the case where the position of rotation diffraction grating 110 is changed in the direction perpendicular to the mirror surface without changing the rotation position of rotation diffraction grating 110. Even with the same rotation position, when the position in the direction perpendicular to the mirror surface is changed, the quantity of light which passes through slit 103 is changed, and the quantity of light incident on PD 107 is changed as illustrated in FIG. 4A and FIG. 4B. With this configuration, a chopper signal can be superimposed on the measurement signal, and noise component can be removed by performing lock-in amplifier detection. As a result, a signal having improved S/N can be obtained, and analysis accuracy is improved. It is to be noted that rotation diffraction grating 110 may be rotated by driving beams 205a and 205b. To be more specific, when beams 205a and 205b are twisted in the same direction, rotation diffraction grating 110 is driven into rotation in a predetermined angle range.

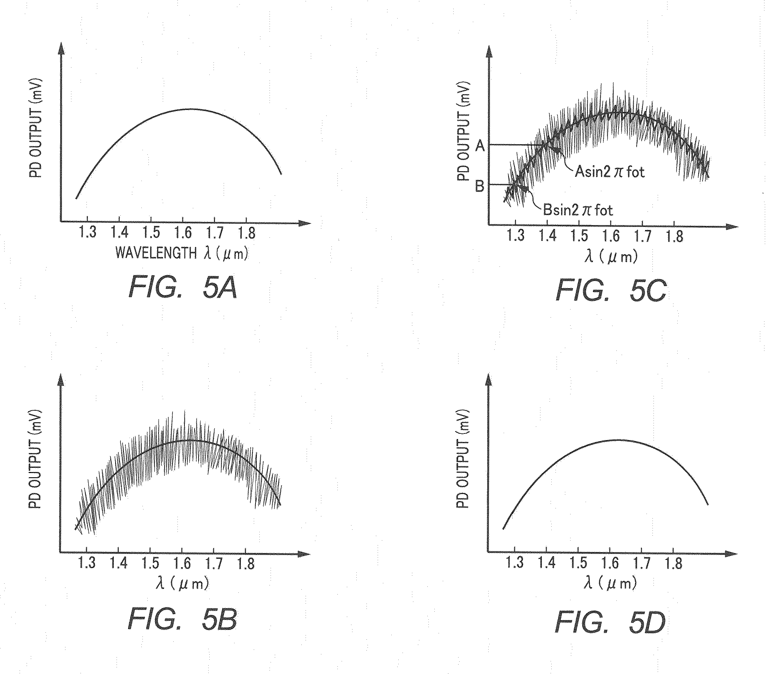

FIGS. 5A to 5D are used for describing lock-in amplifier detection. FIG. 5A illustrates an ideal spectrum without noise. Various frequency noises as illustrated in FIG. 5B are superimposed on an actual measurement signal. FIG. 5C illustrates a spectrum in the case where rotation diffraction grating 110 is driven into high-frequency simple harmonic motion in the direction perpendicular to the mirror surface at frequency f₀. As illustrated in FIG. 5C, a chopper signal of frequency f₀ is superimposed on a measurement signal. FIG. 5D illustrates a measurement signal after a lock-in amplifier detection. Only a signal of frequency f₀ can be taken out as a direct current signal (A and B illustrated in FIG. 5C). With this configuration, the signals having frequencies other than f₀ are removed as noise.

As described, in the present embodiment, measurement light is separated by turning rotation diffraction grating 110, and rotation diffraction grating 110 is driven into high-frequency simple harmonic motion in the direction perpendicular to the mirror surface to improve S/N of the measurement signal. In other words, rotation diffraction grating 110 is biaxially driven in the rotational direction and the direction perpendicular to the mirror surface.

Light composed of λ1 component, light composed of λ2 component, and light composed of λ3 component which are separated in accordance with rotation of rotation diffraction grating 110 sequentially enter sample container 104. The light composed of λ1 component, the light composed of λ2 component, and the light composed of λ3 component which sequentially entered sample container 104 are modulated by measurement target 105 in sample container 104, and thereafter emitted from sample container 104. Here, when passing through measurement target 105, the light composed of λ1 component, the light composed of λ2 component, and the light composed of λ3 component are subjected to respective modulations which are different from each other on the component basis. In this manner, by analyzing the modulations of the wavelength components with computation device 120, the biological component of measurement target 105 can be analyzed.

With the above-mentioned configuration, light is separated using rotation diffraction grating 110. Thus, the optical system can be downsized, and as a result, biological component information measurement device 100 having a small device configuration can be achieved without reducing the measurement accuracy.

In addition, by measuring a urine component with use of biological information measurement device 100, quantitative analysis of the urine component is achieved without requiring reagent or test paper, and the size of the device can be reduced, and therefore, urine inspection can be simply performed at any place. As a result, kidney function or lever function can be determined on a daily basis, which helps health maintenance. Meanwhile, the biological information measurement device of the embodiment of the present invention is applicable to a mobile portable device as well as to a device provided in a toilet.

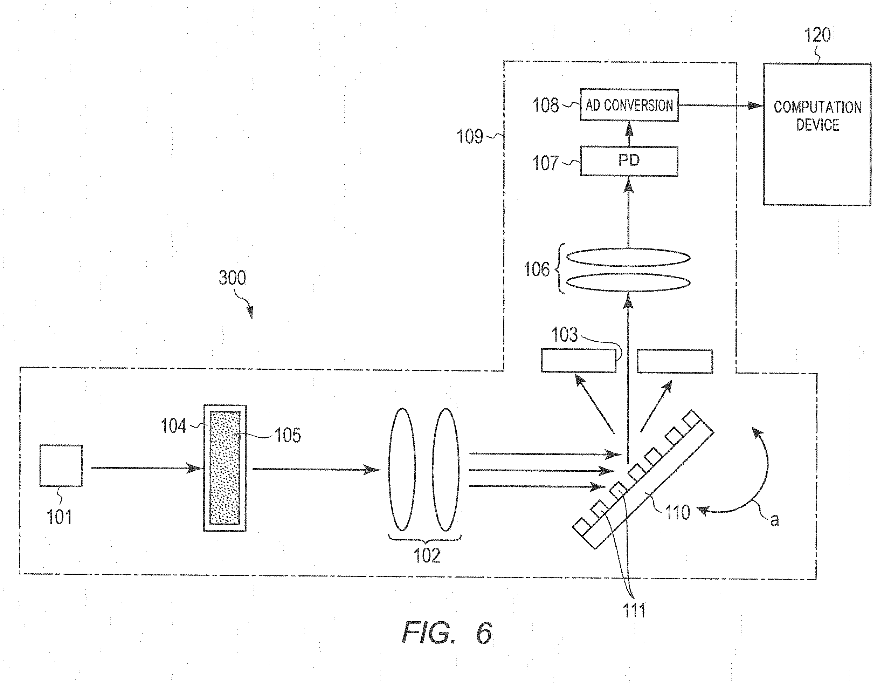

While rotation diffraction grating 110 including the MEMS mirror and diffraction grating 111 formed on the mirror surface of the MEMS mirror is disposed on the optical path from light source 101 to measurement target 105, and rotation diffraction grating 110 separates light from light source 101 such that the light enters measurement target 105 in the configuration illustrated in FIG. 1, the present invention is not limited to this, and the layout illustrated in FIG. 6 may also be employed, for example. In biological component information measurement device 300 of FIG. 6, rotation diffraction grating 110 is disposed on the optical path from measurement target 105 to photodetector (PD) 107, and rotation diffraction grating 110 separates the light having passed through measurement target 105 such that the light is incident on photodetector (PD) 107.

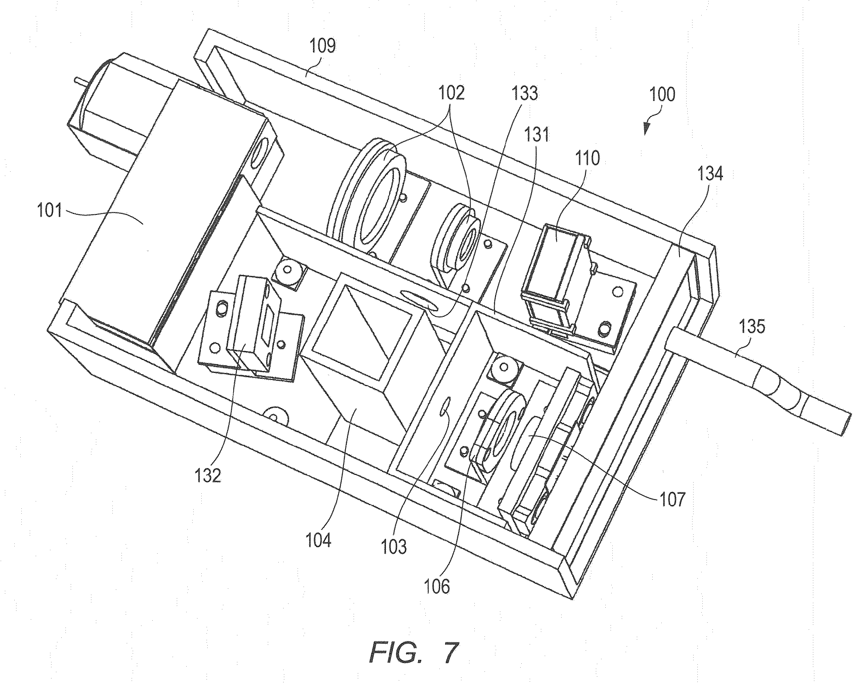

FIG. 1 and FIG. 6 schematically illustrate the biological component information measurement device according to the embodiment. FIG. 7 illustrates the configuration of biological component information measurement device 100 of FIG. 1 in more detail. FIG. 7 is a perspective view of biological component information measurement device 100.

In biological component information measurement device 100 of FIG. 7, in case 109, parting plate 131 separates optical system 102 and rotation diffraction grating (rotation diffraction grating unit) 110, from reflection mirror 132, sample container 104, optical system 106, and PD 107.

With this configuration, the optical systems can be disposed in two lines, and a well-balanced layout can be achieved. In addition, since the optical systems are disposed in two lines, light separating performance can be improved by increasing the length of the light path from rotation diffraction grating 110 to the measurement target while achieving downsizing.

Light emitted from light source (light source unit) 101 is incident on rotation diffraction grating 110 through optical system 102. Optical system 102 is, for example, a collimate system. The light separated by rotation diffraction grating 110 is incident on reflection mirror 132 through opening 133 of parting plate 131. The light reflected by reflection mirror 132 passes through sample container 104 and thereafter is incident on PD 107 through slit 103 and optical system 106. While slit 103 is disposed on the light emission side of sample container 104 in FIG. 7, slit 103 may be disposed on the light incidence side of sample container 104 as illustrated in FIG. 1. Circuit board 134 is provided with a circuit such as AD conversion circuit 108. In addition, circuit board 134 is connected with output cable 135 that is connected the computation device 120 (FIG. 1).

Biological component information measurement device 100 specifically illustrated in FIG. 7 can have a small size that is 10 cm in the longitudinal direction and 5 cm in the width direction, for example.

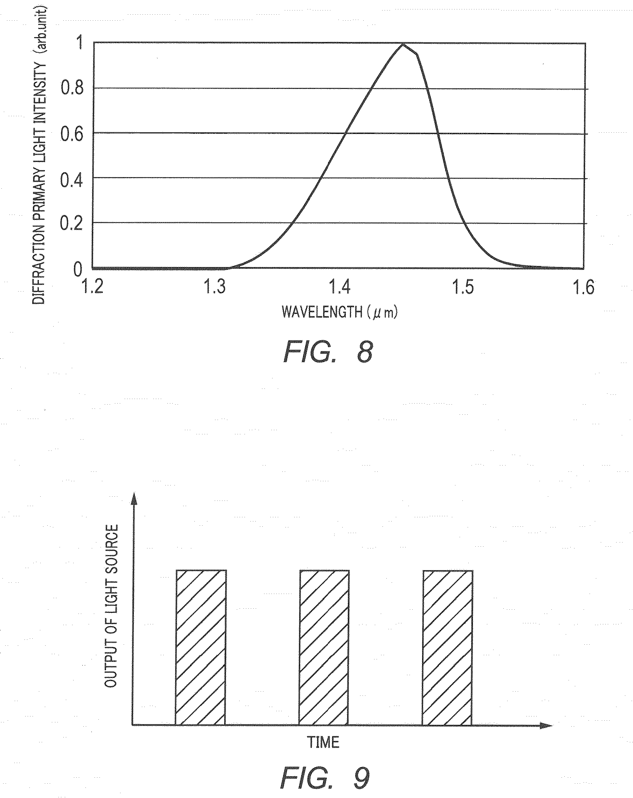

FIG. 8 illustrates a calculated spectrum in the case where LED light whose central wavelength is 1.45 µm is separated by rotation diffraction grating 110 described in the embodiment. From FIG. 8, it was confirmed that a spectrum similar to that of the case where light is separated by use of a line sensor can be obtained. Meanwhile, in the calculation, the angle of light source 101, rotation diffraction grating 110, and PD 107 was set to 50°, and the grating pitch of diffraction grating 111 was set to 2 µm.

While rotation diffraction grating 110 is driven into high-frequency simple harmonic motion in the direction perpendicular to the mirror surface to superimpose a chopper signal on the measurement signal in the above-described embodiment, the way for superimposing the chopper signal is not limited to this. For example, as illustrated in FIG. 9, the chopper signal may be superimposed by pulse driving light source 101. Here, it suffices to set the light emission cycle of light source 101 that is pulse driven to the frequency which is used in the lock-in amplifier.

In addition, while sample container 104 is used as a measurement target placement part for placing measurement target 105 at a predetermined position in the above-described embodiment, the measurement target placement part is not limited to this. In addition, measurement target 105 is not limited to solution such as blood, cultured cells, and urine as long as light can pass through measurement target 105. For example, measurement target 105 may be a section of skin or the like, and in such a case, the measurement target placement part holds the section of skin. In addition, the measurement target placement part may be a space for simply setting the position of measurement target 105.

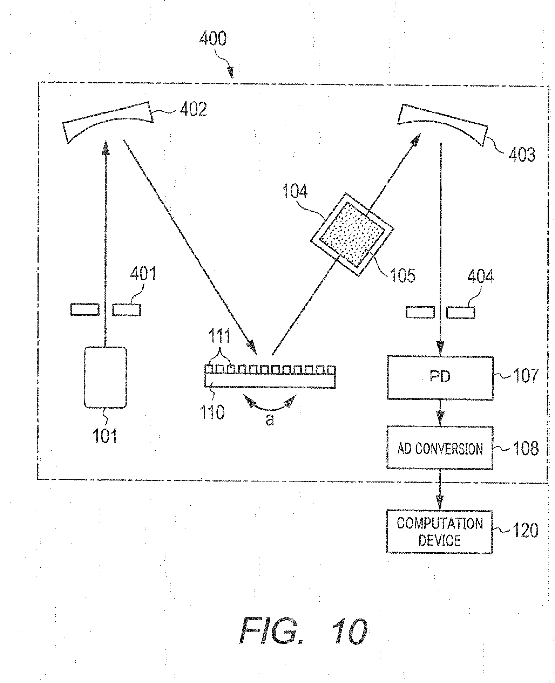

Further, the biological information measurement device of the embodiment of the present invention may employ the layout illustrated in FIG. 10. In FIG. 10, the components corresponding to those of FIG. 1 and FIG. 6 are denoted with the same reference numerals. In biological information measurement device 400 illustrated in FIG. 10, light from light source 101 is incident on collimate mirror 402 through slit 401. Collimate mirror 402 converts the light from the light source into parallel light, and emits the light toward rotation diffraction grating 110. As with the case of embodiment, the light separated by rotation diffraction grating 110 enters sample container 104, and passes through the measurement target in sample container 104 (while being partially absorbed). The light having passed through the measurement target is condensed by condensing mirror 403, and thereafter is incident on PD 107 through slit 404. It is to be noted that sample container 104 may be disposed between collimate mirror 402 and rotation diffraction grating 110. In addition, a plurality of PD 107 and optical components may be provided. Further, collimate mirror 402 and condensing mirror 403 may be a lens system.

Further, while rotation diffraction grating 110 includes a MEMS mirror and a diffraction grating formed on the mirror surface of the MEMS mirror in the above-described embodiment, the present invention is not limited to this. Rotation diffraction grating 110 may include a mirror of an electromagnetic drive type, and a diffraction grating formed on the mirror surface of the mirror of the electromagnetic drive type. In such a configuration, by separating light by driving into rotation the mirror surface of the mirror of the electromagnetic drive type as in the embodiment, an effect similar to that of the embodiment can be obtained.

Rotation diffraction grating 110 is not limited to the diffraction grating of reflection type (mirror type), and may be a diffraction grating of transmission type as long as the diffraction grating can separate light in accordance with the rotation.

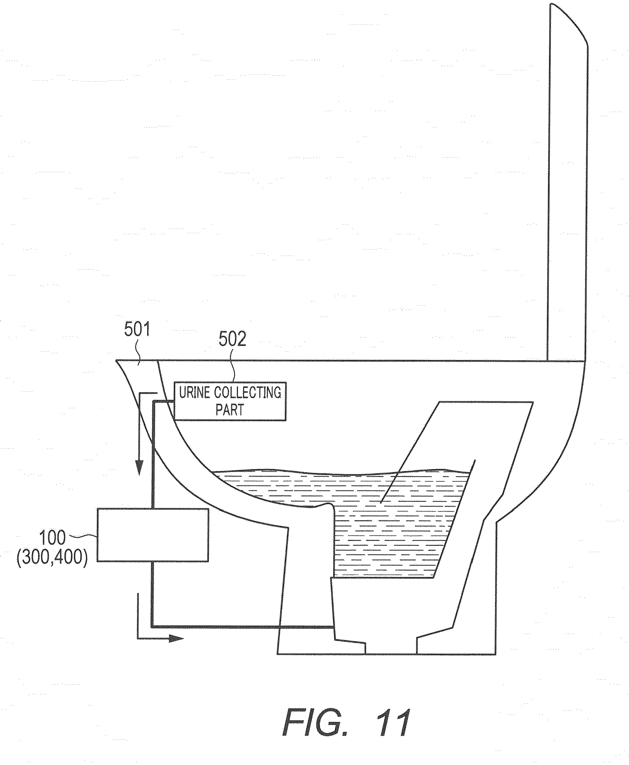

FIG. 11 illustrates an exemplary use of biological information measurement device 100 (300, 400). Urine collecting part 502 is provided at a front portion in toilet 501, and biological information measurement device 100 (300, 400) is provided outside toilet 501. It is to be noted that the position of urine collecting part 502 in toilet 501 is not limited as long as urine can be collected. Urine collected by urine collecting part 502 is sent into sample container 104 of biological information measurement device 100 (300, 400), and the urine component is measured by biological information measurement device 100 (300, 400) with the urine stored therein or the urine flowing therethrough. When the urine component is measured by biological information measurement device 100 (300, 400), the urine in sample container 104 is brought back into toilet 501. According to the embodiment of the present invention, small-sized biological information measurement device 100 (300, 400) can be achieved, and biological information measurement device 100 (300, 400) can be installed in a space where it does not obstruct the user of toilet 501.

It should be understood by those skilled in the art that various modifications, combinations, sub-combinations and alterations may occur depending on design requirements and other factors in so far as they are within the scope of the appended claims or the equivalents thereof. While the invention made by the present inventor has been specifically described based on the preferred embodiments, it is not intended to limit the present invention to the above-mentioned preferred embodiments but the present invention may be further modified within the scope and spirit of the invention defined by the appended claims.

This application is entitled to and claims the benefit of Japanese Patent Application No. 2014-140395 filed on July 8, 2014, and Japanese Patent Application No. 2015-014302 filed on January 28, 2015, the disclosure of which including the specification, drawings and abstract is incorporated herein by reference in its entirety.

### Industrial Applicability

The present invention is applicable to a biological component information measurement device which analyzes a biological component by use of light.

### Reference Signs List

- 100, 300, 400: Biological component information measurement device
- 101: Light source
- 102, 106: Optical system
- 103, 401, 404: Slit
- 104: Sample container
- 105: Measurement target
- 107: Photodetector (PD)
- 108: Analog digital conversion circuit (AD conversion)
- 109: Case
- 110: Rotation diffraction grating
- 111: Diffraction grating
- 120: Computation device
- 131: Parting plate
- 132: Reflection mirror
- 133: Opening
- 134: Circuit board
- 200: MEMS device
- 201: Driving section
- 202: Fixation frame
- 203: Movable frame
- 204 and 205: Beam part
- 204a, 204b, 205a, 205b: Beam
- 402: Collimate mirror
- 403: Condensing mirror

## Claims

1. A biological component information measurement device comprising:
a light source;
a measurement target installation part in which a measurement target is disposed, wherein light from the light source passes through the measurement target and is emitted therefrom;
a photodetector configured to receive light passed through the measurement target; and
a rotation diffraction grating disposed on an optical path from the light source to the measurement target, or an optical path from the measurement target to the photodetector, the rotation diffraction grating being configured to separate the light from the light source such that the light is incident on the measurement target, or separate the light passed through the measurement target such that the light is incident on the photodetector.

2. The biological component information measurement device according to claim 1, wherein:
the rotation diffraction grating includes:
a micro electro mechanical systems (MEMS) mirror; and
a diffraction grating formed on a surface of the MEMS mirror.

3. The biological component information measurement device according to claim 2, wherein:
the rotation diffraction grating rotates such that an incident angle to the surface of the MEMS mirror is changed; and
the rotation diffraction grating oscillates in a direction perpendicular to the surface of the MEMS mirror.

4. The biological component information measurement device according to claim 3, wherein:
the rotation diffraction grating is oscillated in the direction perpendicular to the surface of the MEMS mirror to superimpose a chopper signal on a signal obtained by the photodetector.

5. The biological component information measurement device according to claim 1, wherein the rotation diffraction grating includes:
a mirror of an electromagnetic driving type; and
a diffraction grating formed on a surface of the mirror of the electromagnetic drive type.

6. The biological component information measurement device according to claim 1, wherein the measurement target is urine.
